# EUROPEAN PATENT SPECIFICATION

(11) **EP 3 079 657 B1**
(45) Date of publication and mention of the grant of the patent: **20.03.2019**
(21) Application number: 14809015.2
(22) Date of filing: 08.12.2014
(51) Int. Cl.: A61K 9/06, A61K 47/02, A61K 47/22, A61K 47/38, A61K 31/198, A61K 8/44, A61K 8/20, A61K 33/20, A61K 9/00, A61K 47/18, A61Q 11/00

(54) **PREPARATION FOR PREVENTION AND/OR TREATMENT OF PERI-IMPLANTITIS**
PRÄPARAT ZUR VORBEUGUNG UND/ODER BEHANDLUNG VON PERIIMPLANTITIS
PRÉPARATION POUR LA PRÉVENTION ET/OU LE TRAITEMENT DE LA PÉRI-IMPLANTITE

(30) Priority: 09.12.2013 SE 1351468; 11.04.2014 SE 1450452
(43) Date of publication of application: 19.10.2016
(73) Proprietor: RLS GLOBAL AB, 418 78 Gothenburg (SE)
(72) Inventor: ALMHÖJD, Ulrica, 47537 Bohus-Björkö (SE); FORNELL, Jan, 11538 Stockholm (SE)
(74) Representative: Valea AB
(86) International application number: PCT/EP2014/076909
(87) International publication number: WO 2015/086535

(56) References cited:
- EP-A1- 0 938 282
- WO-A2-2012/172071
- RALF BRGERS ET AL: "The effect of various topical peri-implantitis antiseptics on,, and", ARCHIVES OF ORAL BIOLOGY, PERGAMON PRESS, OXFORD, GB, vol. 57, no. 7, 28 January 2012 (2012-01-28), pages 940-947, XP028499457, ISSN: 0003-9969, DOI: 10.1016/J.ARCHORALBIO.2012.01.015 [retrieved on 2012-02-04]

## Description

### TECHNICAL FIELD

The present invention relates to a kit of parts and/or a preparation for use in prevention and/or treatment of peri-implantitis.

### BACKGROUND

Periodontal diseases and aplasia are common reasons why some people lack one or more teeth. For many years the only appliance for missing teeth was bridges. Bridges are composed of one or more artificial teeth which are attached firmly to neighboring natural teeth. This technique is excellent for replacing one or two teeth and has the advantage of being stable and not removable. Bridges are useful for replacing only the missing teeth, but cannot replace any missing bone or gum tissue. Their main disadvantage is that the teeth necessary to support the bridge may not be strong enough to bear any additional load, or they may not be in the right places. In addition, the aggressive grinding on the existing teeth which is necessary to attach the bridge is not desirable. Still a disadvantage of bridges is that thorough cleaning around the bridge is often difficult making it hard to keep good oral hygiene.

Dental implants are an attractive alternative to bridges and have been used for more than 20 years. The dental implant is a surgical device used to replace the one or more missing teeth. The dental implant comprises a part that is fused to the bone by so-called osseointegration. The dental implant may further support a crown or bridge. Thus, the dental implant may be seen as a way of providing an artificial tooth root to which a crown may be attached.

There are many advantages associated with dental implants. For instance, use of dental implants usually provides a good appearance and good comfort. Further, dental implants feel and function like a person's own teeth.

For a long time it was believed that dental implants were associated with no serious side effects. However, it is now realized that dental implants may cause diseases, infections and/or inflammations. Among these dental implant-related diseases, infections and/or inflammations so-called peri-implantitis and oral mucositis associated with a dental implant account for a large number of cases.

Peri-implantitis has been defined as a localized lesion involving bone loss around an osseointegrated implant. Peri-implantitis is a serious complication, and it is believed to occur in a large number of patients with dental implants. It has been reported that for patients having a dental implant one out of ten or even more suffer from peri-implantitis. Thus, it is clear that peri-implantitis is an increasing problem in modern dentistry.

Oral mucositis associated with a dental implant, which may also be denominated oral mucositis or peri-implant mucositis, is a reversible inflammatory change of the soft tissues surrounding the dental implant without concomitant bone loss. The prevalence of oral mucositis is difficult to estimate, but is believed to be higher than that of peri-implantitis. Generally, oral mucositis is a stage preceding peri-implantitis.

Features that contribute to making prevention and/or treatment of peri-implantitis difficult are the rough surface and/or the threads of the dental implants. The rough surface and the threads increase the dental implant surface area thereby improving osseointegration and healing after installation of the dental implant. However, in case of infection and/or microbial colonization a large dental implant surface area is a disadvantage since it allows for microorganisms to harbour in places that are difficult to see visually and/or reach with instruments and/or preparations thereby making microorganism eradication and killing difficult.

In Oral Health Journal, August 2013, Mark Nicolucci describes current treatments of peri-implantitis. It is disclosed that treatment of peri-implantitis may be approached like treatment of periodontitis. However, periodontitis and peri-implantitis do not respond to treatment similarly. Peri-implantitis lesions are said not to respond well to improved oral hygiene and professional cleanings as is highly effective with periodontitis.

Attempts to treat infected implant surfaces have been made. In the abstract of Arch Oral Biol. 2012 Jul;57(7):940-7 it is disclosed that sodium hypochlorite was effective when tested as an antimicrobial agent on biofilm on titan specimens, but was not recommended for the topical disinfection and detoxification of infected implant surfaces due to possible toxicity and lack of broad-spectrum antimicrobial effect.

WO 2012/172071 discloses a kit of parts for detection and removal of carious dentin tissue comprising: (I) one or more compounds being a hydrazine derivative, and (ii) means for chemical treatment of carious dentin tissue. There is also disclosed a preparation containing an active, caries-dissolving two-component liquid in the form of a first active, caries-dissolving component and a second component which reduces the aggressiveness of the active component to mucuous membranes, a viscosity increasing substance and one or more compounds being a hydrazine derivative.

EP0938282 discloses a preparation for use in chemical-mechanical treatment of caries in the form of a two-component caries dissolving liquid in which one of the components consists of sodium hypochlorite and the other component of a substance with the ability to reduce the aggressive influence of sodium hypochlorite on mucous membranes. The other component is a gel component which consists of a gel substance without any amino acids or a gel substance mixed with one or more amino acids.

Prevention and/or treatment of peri-implantitis are commonly carried out in specialist clinics such as dentist specialist clinics. Frequently, the treatment is followed up in a generalist clinic. It would be desirable, however, if at least part of the prevention and/or treatment could take place in a generalist clinic or even in a patient's home since this is often more convenient for the patient. In particular, prevention of peri-implantitis would be desirable thereby avoiding or minimizing peri-implantitis.

Thus, there exists a need for methods allowing for prevention and/or treatment of peri-implantitis as well as for preparations for use in said methods.

It is an object of the present invention to fulfill said need and/or to overcome or at least mitigate some of the disadvantages associated with the prior art.

### DESCRIPTION

The disclosure provides a kit of parts for use in the treatment and/or prevention of peri-implantitis according to independent claim 1. Further, the disclosure provides a treatment preparation for use in treatment and/or prevention of peri-implantitis according to independent claim 16. Further embodiments are set out in the dependent claims and in the following description.

Accordingly, there is provided a kit of parts for use in the prevention and/or treatment of peri-implantitis. The kit of parts comprises
a) a first aqueous component comprising one or more amino acids, and
b) a second aqueous component comprising an active halogen compound. The pH of the first component and/or the second component may be from 9 to 11.5.
The treatment of peri-implantitis may be surgical, i.e. surgical treatment of peri-implantitis.

The kit of parts may further comprise instructions for use. The instructions may involve instructions for mixing the first aqueous component with the second aqueous component into a treatment preparation, how to apply the treatment preparation and/or how often the treatment preparation should be applied. The instructions may also include instructions for adding further components such as one or more compounds being a hydrazine derivative. The hydrazine derivative may be a compound of formula (1) as described herein.

As described herein, peri-implantitis is understood to be a localized lesion involving bone loss around an osseointegrated dental implant. Further, the peri-implant pockets and/or tissues surrounding the dental implant may be swollen and/or involved in inflammation and/or infection. Oral mucositis is understood to be a reversible inflammatory change of the soft tissues surrounding the dental implant without concomitant bone loss. Generally, peri-implant mucositis is a stage preceding peri-implantitis. In this document, the expressions "oral mucositis", "oral mucositis associated with a dental implant" and "peri-implant mucositis" are used interchangeably.

The term "active halogen compound" herein refers to a halogen in the form of an ion, gas, salt or hypohalogenite of a halogen.

The two components of the kit of parts are intended to be mixed together and thereby form a treatment preparation for use in the prevention and/or treatment of peri-implantitis.

The first component reduces the aggressiveness of the active halogen compound to living tissues. The treatment preparation formed from the two components comprises one or more amino acids that provide less aggressive and toxic means for treating peri-implantitis and/or oral mucositis. The treatment with the treatment preparation utilizes the two liquid components which when combined to the treatment preparation and applied on a site of treatment such as a dental implant surface and/or lesions associated with peri-implantitis lead to a chemical reaction which is believed to result in primary halogenated amines in the form of (primary) halogenated amino acids such as primary chloroamino acids. By primary chloroamino acids is intended amino acids in which the amine carries one chlorine atom. The treatment disclosed herein is believed to utilize this chemical reaction.

The kit of parts for use in prevention of peri-implantitis described herein may be applied by administration of the first and second components once or several times. Prior to administration, the first and second components are mixed to form a treatment preparation. Thus, the first and second components of the kit of parts may be administered to a treatment site repeatedly. For instance, examination of a treatment site such as tissues and/or peri-implant pockets will reveal if it is suitable to add the first and second components. The examination may be visual inspection. The examination may be made with respect to bleeding on probing, the degree of swelling of the tissues, colour of the tissues etc. The skilled person such as a dentist will be able to determine which time interval between treatments is appropriate, i.e. how often the first and second components should be applied. As an example, the time interval between treatments may be one, two, three, four, five, six weeks or more.

As used herein, the term "treatment" refers to treatment of peri-implantitis aiming at removal, eradication and/or killing of microorganisms such as bacteria in order to allow for osseointegration and healing. The treatment thus results in improved osseointegration and healing. As used herein, the term "osseointegration" is understood to mean the formation of a direct contact between the dental implant surface and bone. The term "prevention" refers to a measure taken in order to minimize and/or avoid peri-implantitis. Prevention may take place at a stage preceding peri-implantitis. Oral mucositisis is a stage preceding peri-implantitis, and treatment of oral mucositis is thus a way of preventing peri-implantitis.

It is particularly desirable to prevent peri-implantitis. Significantly, it has been found by the inventors of the present invention that prevention may be carried out using the treatment preparation described herein. As shown in the Examples, prevention using the treatment preparation Perisolv® or Perio+® was successful in preventing peri-implantitis. Similarly, oral mucositis could be treated using Perisolv® or Perio+®.

The uses of chloroamines as is known in the art commonly rely on the use of reactions that are subsequent to the first reaction, i.e. the reaction in which primary halogenated amines are formed, and in which the preparation should have a low pH, i.e. just below neutral pH such as pH 6. These subsequent reactions result in dihalogenated amines, i.e. amines in which the nitrogen carries two chlorine atoms. The pH is then normally lower than a neutral pH, which is contrary to the present treatment and/or prevention with the treatment preparation disclosed herein wherein the pH is high and basic.

After being mixed the components will also have low impact on the environment, as the mixed components will decompose into salts, gases and water in nature. Attention is drawn to the fact the concentrations of halogen such as chlorine used in the components and treatment preparations are lower than the amounts of chlorine used in chlorination of water for purification thereof or in normal house hold bleach product containing chlorine.

The pH of the first and/or second component is from 9 to 11.5, but specifically may be from 9 to 11, or from 9.5 to 11.5, or from 10 to 11.5, or from 10.5 to 11.5, or from 11 to 11.5, or from 9 to 10.5. The treatment preparation may have antibacterial effects making it useful for the treatment and/or prevention of dental implant-related diseases, infections and inflammations such as peri-implantitis and oral mucositis.

The active halogen compound is an active chlorine compound selected from the group consisting of Cl₂, hypochlorite, chlorite, chlorate, perchlorate and/or a hypochlorite compound. Further, the active chlorine compound may be Cl₂, hypochlorite or a hypochlorite compound. Further, the active chlorine compound may be hypochlorite or a hypochlorite compound.

The ion compounds may be in the form of sodium, calcium, lithium and/or potassium form, e.g. the hypochlorite compound may be sodium hypochlorite, calcium hypochlorite, lithium hypochlorite and/or potassium hypochlorite.

The amount of the active halogen compound in the second component may be 0.5-5, 0.5-3, or 1-2 % (by weight).

If nothing else is mentioned, percentage by weight herein means to refer to the weight of a compound or the like relative the total amount of the mentioned component or the like comprising the compound. Percentage by weight is also denominated weight% or wt%. In this document, the expressions "% (by weight)", "weight%" and "wt%" are used interchangeably. Alternatively, the concentration may be expressed as weight/weight % (w/w%). Since the first and second components are aqueous compositions the concentration may also be expressed as density using w/v, i.e. weight per volume.

Combining the second component including a halogen compound such as hypochlorite with the first component including one or more amino acids such as glutamic acid, leucine and lysine is effective for prevention and/or treatment peri-implantitis. It is also effective for treating oral mucositis.

Advantageously, it has been found that use of a treatment preparation as described herein will not adversely affect the surface of a titanium implant. This is a significant benefit, since the dental implant surface is generally designed to facilitate osseointegration and therefore should not be changed or damaged. Thus, use of the treatment preparation as described herein will have no or minimal negative impact on the osseointegration process of the implant.
A used herein, amino acids are organic compounds made from amine (-NH₂) and carboxylic acid (-COOH) functional groups, along with a side-chain specific to each amino acid.

The one or more amino acids is selected from the group consisting of alanine, arginine, asparagine, aspartic acid, glutamic acid, glutamine, isoleucine, leucine, lysine and/or valine.

These amino acids have been identified to be of the kind to form primary halogenated amino acids such as primary chloroamino acids. The pK values and/or properties of the side chains are such that the primary halogenated amino acids may be formed and uses thereof also cause low risk, if any, of adverse effects on a subject such as a patient.

The one or more amino acids may comprise or consist of glutamic acid, leucine and lysine.

According to an embodiment, the first component may comprise 0.1-1 % (by weight) of said one or amino acids.

The amino acid concentration in the first component may be 0.1-1%, 0.4-1.0 % or 0.5-1 % (by weight), or 0.4-0.8 % or 0.5-0.8 % (by weight). The amino acid composition may contain a mixture of amino acids mixed in a relation by weight between each amino acid of 2:1 to 1:2, preferably 1:1.

The two components may be mixed in the volume ratios of 1:2 to 2:1, preferably 1:1, thereby forming the treatment preparation for use in the treatment and/or prevention of an implant-related disease, infection and/or inflammation such as peri-implantitis and/or oral mucositis. At 20 degrees Celsius and a pressure of 101325 Pa, and within 30 seconds after the components have been mixed together, the treatment preparation has a pH of from 9 to 11.5, or from 9 to 11, or from 9.5 to 11.5, or from 10 to 11.5, or from 10.5 to 11.5, or from 11 to 11.5, or from 9 to 10.5, or from 9.5 to 10.5.

The treatment preparation described herein may be applied once or several times during the same treatment session and/or during separate treatment sessions. Thus, the treatment preparation may be administrated to a treatment site repeatedly. Before a new treatment session takes place, examination of a treatment site such as tissues and/or peri-implant pockets will reveal if it is suitable to add the treatment preparation. The examination may be visual inspection. The examination may be made with respect to bleeding on probing, the degree of swelling of the tissues, colour of the tissues etc. The skilled person such as a dentist or a dental hygienist will be able to determine which time interval between treatment sessions is appropriate, i.e. how often the treatment preparation should be applied. As an example, the time interval between treatment sessions may be one, two, three, four, five, six weeks or more. Generally, treatment of peri-implantitis with the treatment preparation described herein takes place only once whereas prevention of peri-implantitis may comprise application of the treatment preparation during one or several treatment sessions.

The treatment preparation described herein facilitates removal of undesired matter thereby shortening the treatment time. Preferably, the steps are repeated during the treatment session so that the treatment preparation is applied several times.

In this way there is provided a way of securing that the reaction forming the primary halogenated amino acids have the proper reaction condition to occur on the treatment site. The treatment site may be a dental implant, a peri-implant pocket, a peri-implantitis lesion and/or tissues associated with peri-implantitis or oral mucositis. Thus, the provision of a kit of parts provides means to control that the desired reaction occurs on the treatment site.

The use may involve applying the treatment preparation before or during the appearance of gas bubbles from the treatment preparation. If an active chlorine compound is used, a smell of chlorine also appears along with the gas bubble formation. In this way, the occurrence of the proper reaction is secured.

The first aqueous component may further comprise a gel substance.

The gel substance provides moist keeping means to keep a moist environment for the treatment site, and in particular the gel substance reduces evaporation from the aqueous treatment preparation prepared from the first and second components, when applied to the treatment site. Furthermore, the gel substance also provides a proper consistency to the treatment preparation prepared from the two components.

A treatment preparation provided with a gel substance and having the "high" basic pH provides the use for effective treatment and/or prevention as mentioned herein above, while providing low aggressiveness to a treatment site.

The gel substance may comprise or consist of polyethylene glycol (PEG), and/or carboxymethyl cellulose and/or a polysaccharide substance or a salt thereof, such as sodium carboxymethyl cellulose (Na-CMC).

Such a substance provides said consistency and acts as a moist keeping substance preventing said evaporation from the treatment preparation and treatment site.

The first component may comprise 2-4 % (by weight) gel substance.

The first component may further comprise TiO₂ and/or NaCl.

The addition of NaCl adds to the active chlorine compound content present in the first component and treatment preparation. NaCl also has an antibacterial effect as such.

The components may be the ones sold under the trademark Carisolv® , Perisolv® or Perio+®(RLS Global AB).

Carisolv® ,Perisolv® or Perio+® may have the compositons indicated in Table I. In other words, Carisolv® ,Perisolv® or Perio+® may have the following compositions.

The preparation Carisolv® is an aqueous composition comprising a first aqueous component comprising a mixture of glutamic acid, leucine and lysine (0.1-1 wt%), NaCl (0.3-0.6 wt%) and sodium carboxymethyl cellulose gel (2-4 wt%), and a second aqueous component comprising NaOCI (1-2 wt%). The first component and the second component may be added in an amount providing a pH from 9 to 11.5.

The preparation Periosolv® or Perio+® is an aqueous composition comprising a first aqueous component comprising a mixture of glutamic acid, leucine and lysine (0.1-1 wt %), TiO₂ (0.03-0.1 wt%), NaCl (0.3-0.6% wt%) and sodium carboxymethyl cellulose gel (2-4 wt%), and a second aqueous component comprising NaOCI (1-2 wt%). The first component and the second component may be added in an amount providing a pH from 9 to 11.5.

The clinical effectiveness with respect to prevention and/or treatment of peri-implantitis may be further enhanced by subjecting the treatment preparation as described herein, wherein said treatment preparation includes TiO₂, to irradiation from 10 to 700 nm, or from 200 to 400 nm or from 400 to 700 nm. In this document, the term "nm" stands for nanometer. While not wishing to be bound by any specific theory, it is believed that this enhanced clinical effect may be due to the formation of singlet oxygen. Previously, it has been reported that singlet oxygen produced by irradiation of TiO₂ may be involved in oral bacterial disinfection (J. Clin. Biochem. Nutr., September 2012, Vol. 51, No. 2, pages 128-131.)

The kit of parts may therefore further comprise an instrument able to operate at wavelengths such as ultraviolet (uv) wavelengths. The instrument may be operated at from 10 to 700 nm, or from 200 to 400 nm or from 400 to 700 nm, and may be used to irradiate the treatment preparation described herein

A major difficulty when cleaning and/or treating dental implants is to remove all or substantially all undesired matter such as infected tissue on the implant surface. Detection of infected tissue may be facilitated by staining of said infected tissue using a hydrazine derivative. Advantageously, staining allows visual detection of undesired matter such as infected tissue on a dental implant surface.

The kit of parts may therefore further comprise one or more hydrazine compounds of formula (I):

RNHNH₂ (I)

The R group of the hydrazine compound RNHNH₂ is a chromophore or forms a chromophore with NHNH₂.

In this document, the term "chromphore" means the part of the molecule resulting in its colour. The colour arises when that part of the molecule absorbs certain wavelengths of light such as visible light and transmits or reflects others. In this document, visible light is defined as electromagnetic radiation having a wavelength in the range of 380 nm to 750 nm. Detection may be made without or with the aid of an optical instrument.

It is to be understood that the hydrazine derivative of formula (I) may be, for instance, a carbazide or a hydrazide.

Examples of hydrazine derivatives of formula (I) that may be used include compounds of formula (Ia), (Ib), (Ic) and (Id) below. 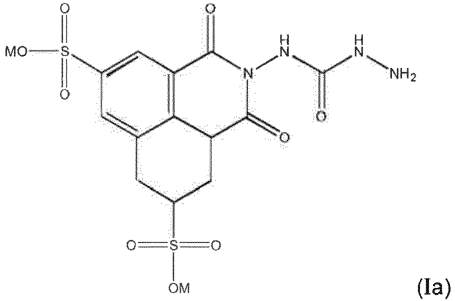 wherein M represents a monovalent metal ion selected from Li⁺, K⁺ and Na⁺. When M is K⁺ the compound of formula (Ia) is denominated Lucifer Yellow. 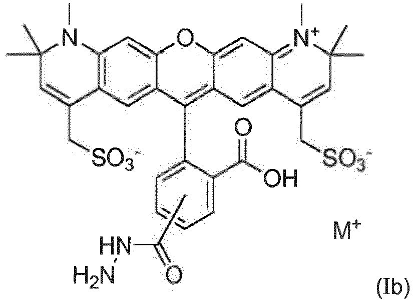 wherein M⁺ represents a monovalent metal ion selected from Li⁺, K⁺ and Na⁺.
The trade name of the compound of formula (Ib) when M⁺ is Na⁺ is Alexa Fluor® 594 hydrazide sodium salt.

The chemical name for the compound of formula (Ib) is 6-(2-carboxy-5-(hydrazinecarbonyl)phenyl)-1,2,2,10,10,11-hexamethyl-2,10-dihydro-1H-pyrano[3,2-g]diquinoline-11-ium-4,8-diyl)methanesulfonate. 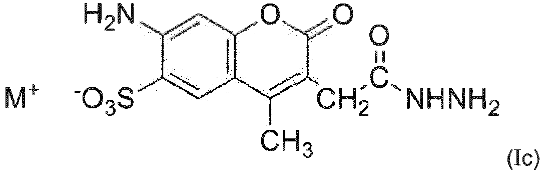 wherein M⁺ represents a monovalent metal ion selected from Li⁺, K⁺ and Na⁺.

The trade name of the compound of formula (Ic) when M⁺ is Na⁺ is Alexa 350.

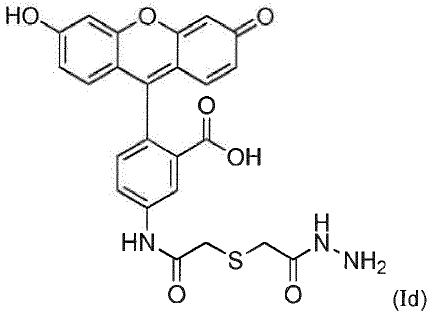

The trade name of the compound of formula (Id) is 5-(((2-(carbohydrazino)methyl)thio)acetyl)aminofluorescein, whereas the chemical name suggested by Chemdraw (CS Chemdraw Ultra, Cambridge Soft, USA) for the compound (Id) is 5-(2-2-hydrazinyl-2-oxoethylthio)acetamido)-2-(3-hydroxy-6-oxo-6*H*-xanthen-9-yl) benzoic acid.

It will be appreciated that the hydrazine derivative mentioned herein may be mixed with one or more other hydrazine derivatives. Further, the hydrazine derivative may be mixed with other dyes such as Patent Blue V. Patent Blue may exist as its calcium salt and has CAS number 3536-49-0.

Further examples of of hydrazine derivatives include HiLyte Fluor™ 488 hydrazide, HiLyte Fluor™ 555 hydrazide, HiLyte Fluor™ 594 hydrazide, HiLyte Fluor™ 647 hydrazide, HiLyte Fluor™ 680 hydrazide

In order to ensure that all or essentially all undesired matter such as infected tissue is removed, eradicated and/or killed a solution of a hydrazine derivative may be added to the implant thereby staining any present infected tissue. If infected tissue is present, they may easily be detected by staining and subsequently removed. The infected tissue is believed to contain bacteria. While not wishing to be bound by any specific theory, it is believed that bacteria contain ester groups and that staining may take place upon reaction of these ester groups with the hydrazine derivative.

The hydrazine derivative may be added to the implant surface followed by addition of the treatment preparation as described herein. For instance, an aqueous solution of the hydrazine derivative may be provided to the implant surface followed by addition of the treatment preparation described herein. Rinsing with an aqueous solution may take place after addition of the hydrazine derivative to the implant surface. Alternatively, the hydrazine derivative may be mixed with the treatment preparation. In the latter case, staining may be visualized after rinsing and then further treatment preparation may be added to the stained parts of the implant surface. Removal of the infected tissue may be further improved by mechanical treatment of the implant surface. For this purpose, a dental scrape instrument may be used.

There is also provided a treatment preparation for use in prevention and/or treatment of peri-implantitis, wherein the treatment preparation is obtainable by mixing a first and a second component as described herein. The first aqueous component and the second aqueous component may be mixed in a volume ratio of 1:2 to 2:1 or in a volume ratio of 1:1. The prevention may include treatment of peri-mucositis.

Such a treatment preparation has shown the desired effects in uses described herein. The treatment preparation may have a pH of 9 to 11.5 within 30 seconds after the components have been mixed together (at 20 degrees Celsius and a pressure of 101325 Pa). The pH is from 9 to 11.5, but specifically may be from 9 to 11 or from 9 to 10.5, or from 9,5 to 10.5, or from 9.5 to 11.5, or from 10 to 11.5, or from 10.5 to 11.5, or from 11 to 11.5.
This provides the means for the proper reaction to occur on the treatment site such as an implant surface, peri-implant pockets and/or tissue associated with the dental implant described herein, while providing all the above-mentioned advantages.

As mentioned herein, the use of a treatment preparation may involve applying the treatment preparation to a treatment site. The treatment preparation may be applied before or during formation of gas bubbles appearing from the treatment preparation. If an active chlorine compound is used, a smell of chlorine also appears along with the gas bubble formation. In this way, the occurrence of the proper reaction is secured.

### BRIEF DESCRIPTION OF THE DRAWINGS

Fig. 1 shows an X-ray photograph of a dental implant involved in peri-implantitis. The threads have been filled in to enhance visibility.
Fig. 2 is an X-ray photograph of the healed dental implant after one year. In Fig. 2 the threads have been filled in to enhance visibility.

The invention is further illustrated by the following non-limitative Examples.

### EXAMPLES

### Example 1

In Table I an example of the content of the two components as part of a kit for providing the treatment preparation is provided. The contents correspond in large to the components and preparations sold under the trademarks Carisolv® and Perisolv®.

| **Table I.** | | |
|---|---|---|
| Contents of component 1 and component 2 to be mixed with each other just before application on a treatment site of a subject. (water is present up to 100 % by weight) | | |
| | Component 1 (% by weight) | Component 2 (% by weight) |
| NaOCl | - | ≤1-2 |
| Amino acids Lys, Glu and Leu | 0.4-0.8* | - |
| TiO₂ | 0/0.03-0.1 | - |
| NaCl | 0.3-0.6 | - |
| Na-CMC (high viscosity/medium viscosity) | 2-4 | - |
| NaOH | Added in an amount providing a pH from 9 to 11.5 | Added in an amount providing a pH from 9 to 11.5 |

| | | |
|---|---|---|
| *total amount of amino acids, the amino acids being present in a relation by weight of about 1:1:1. | | |

In Example 2 and Example 3 below the numbering of the teeth follows the Universal Numbering System. The treatments were performed by an experienced dentist consultant in periodontology. The preparation Perisolv ® was freshly prepared prior to use.

### Example 2. Surgical treatment of peri-implantitis.

The patient was a male born in 1943 who was on medication for high blood pressure and high levels of cholesterol. The patient was a nonsmoker. He had a dental implant in position 25. The dental implant was from the company Straumann, with seven threads (4.1 x 10 mm). In this document, the term "mm" stands for millimeter. The dental implant had been installed in 2007.

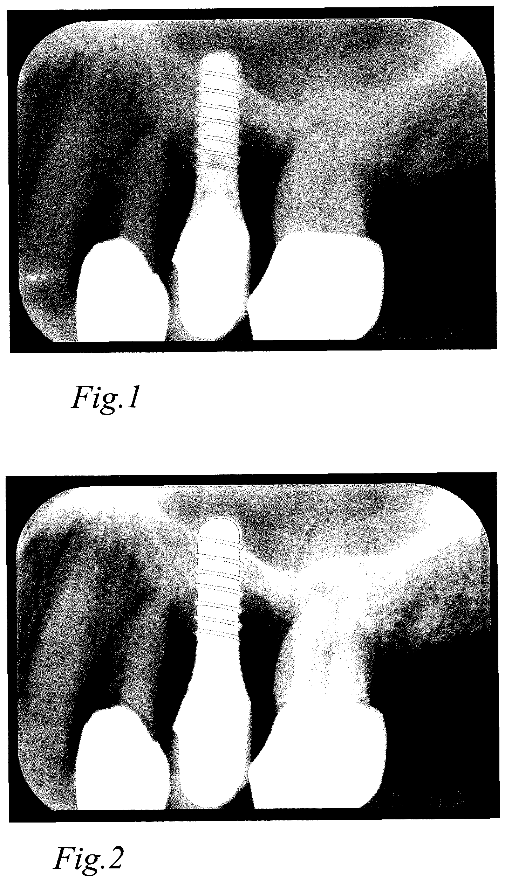

The patient was diagnosed with peri-implantitis in 2009. Clinical investigation revealed that an infection was present. X-ray analysis showed that the dental implant was shaded, which is interpreted as an infection. Fig. 1 shows an X-ray photograph of the dental implant involved in the peri-implantitis. In Fig. 1 the threads have been filled in to enhance visibility. The patient was treated as follows.

The patient was operated. Anesthetics with xylocaine-adrenaline was given to the patient. Buccal and palatinal muco-periost flaps were raised. Perisolv ® was applied onto the surface of the dental implant and left for 30 seconds. Mechanical cleaning was then performed using an ultrasonic device with curettage of granulation tissue. Rinsing with water took place continuously during the mechanical cleaning. Perisolv ® was then applied again onto the dental implant surface and surrounding tissues. Then, ultrasonic treatment in combination with rinsing with water took place. Due to the bone loss associated with the peri-implantitis the top four threads of the dental implant were not connected to bone. Bone ceramics from the company Straumann was therefore added around the dental implant in such a way that it was filled into the bone defect. Flap closure was performed using a suture closure procedure.

The patient was prescribed the antibiotic Azytromax, 500 mg, 6 tablets of which 2 should be taken the first day and thereafter one tablet per day. The patient was also prescribed mouth rinsing with chlorhexidine twice a day for a week.

The patient came back to the clinic for check-ups and X-ray analysis one week, three months, one year, two years and four years later. Complete healing of the peri-implantitis was observed one year after the operation had taken place, and the healing still remained after four years. All treads of the dental implant were covered by bone. X-ray analysis showed that the dental implant was no longer shaded, which means that complete healing has taken place. No inflammation was noticed. Fig. 2 is an X-ray photograph of the healed dental implant after one year. In Fig. 2 the threads have been filled in to enhance visibility.

### Example 3. Prevention of peri-implantitis and treatment of oral mucositis.

The patient was a female born in 1949. The patient was a non-smoker. She was healthy, lacked tooth 11, and in 1999 she had a dental implant installed in position 11.
The patient payed regular visit to a dental hygienist and during one of the routine check-ups inflammation around the dental implant was found, i.e. oral mucositis was diagnosed. The oral mucosa around the implant was swollen, pus was present and there was bleeding on probing. The peri-implant pocket depth around the dental implant was considered to be pathologic. X-ray analysis showed that the bone around the dental implant was slightly lower than normal. The bone lowering did not reach the first implant thread, though. The dentist examined the patient and interpreted the situation as a stage preceding peri-implantitis, said stage involving mucositis and slight bone resorption. The dentist then treated the patient as described below.

Perisolv ® was applied for 30 seconds in the peri-implant pocket surrounding the dental implant. Mechanical treatment using an ultrasonic device and curettage of granulation tissue took place. The procedure was repeated once more. At the end, rinsing with water took place. The treatment took approximately 15 minutes. Three weeks later the patient came for a check-up. The tissue surrounding the dental implant looked healthy and normal. X-ray analysis showed that the bone close to the dental implant had started to heal.

It was concluded that the treatment with Perisolv ® was successful in preventing peri-implantitis and in treating oral mucositis.

### Example 4

This experiment was performed in order to investigate if remnants of tissue on an implant neck could be detected by staining with a hydrazine derivative. The hydrazine derivative Lucifer Yellow was chosen, since it will exhibit fluorescence when irradiated with a suitable lamp.

An extracted dental implant from a patient suffering from peri-implantitis was received. The patient had received no treatment for peri-implantitis and the implant neck was thus provided with tissue from the patient. The implant was subjected to an aqueous solution of the hydrazine derivative Lucifer Yellow in a concentration of 15 mM. In this document, mM stands for millimolar, i.e. millimoles per liter. After rinsing with water the implant neck was subjected to irradiation from an ordinary dental lamp operating between 400 and 500 nm in order to see if fluorescence could be detected. Fluorescence was observed. A photograph was taken under a microscope, and it was concluded that tissue remained on the implant neck. The tissue was then cleaned with Carisolv ® together with a brush. The cleaning was followed by rinsing with water. Visual inspection with the aid of the lamp revealed staining of some parts. Thus, some tissue remained on the implant neck. Cleaning was repeated with Carisolv ® until no fluorescence was detected.

It was concluded that hydrazine derivatives allowed for detection of tissue on a dental implant.

## Claims

1. A kit of parts for use in treatment and/or prevention of peri-implantitis, wherein the kit of parts comprises
a. a first aqueous component comprising one or more amino acids selected from the group consisting of alanine, arginine, asparagine, aspartic acid, glutamic acid, glutamine, isoleucine, leucine, lysine and/or valine, and
b. a second aqueous component comprising an active chlorine compound selected from the group consisting of Cl₂, hypochlorite, chlorite, chlorate, perchlorate and/or a hypochlorite compound,
wherein the first aqueous component and/or the second aqueous component has a pH from 9 to 11.5,and
wherein a treatment preparation obtainable by mixing said first aqueous component and said second aqueous component at 20 degrees Celsius and at a pressure of 101325 Pa within 30 seconds after mixing has a pH from 9 to 11.5.

2. The kit of parts for use according to claim 1, wherein the kit of parts is for use in the treatment of peri-implantitis.

3. The kit of parts for use according to claim 2, wherein the treatment of peri-implantitis comprises or consists of surgical treatment of peri-implantitis.

4. The kit of parts for use according to claim 1, wherein the kit of parts is for use in prevention of peri-implantitis.

5. The kit of parts for use according to claim 4, wherein the prevention of peri-implantitis comprises or consists of treatment of oral mucositis.

6. The kit of parts for use according to any one of the preceding claims, wherein the amount of the active chlorine compound in the second aqueous component is 0.5-5, 0.5-3, or 1-2 wt%.

7. The kit of parts for use according to any one of the preceding claims, wherein said one or more amino acids comprise or consist of glutamic acid, leucine and lysine.

8. The kit of parts for use according to any one of the preceding claims, wherein the first aqueous component comprises 0.1-1 wt% of said one or more amino acids.

9. The kit of parts for use according to any one of the preceding claims, wherein the first aqueous component further comprises a gel substance.

10. The kit of parts for use according to claim 9, wherein the gel substance comprises or is polyethylene glycol (PEG), and/or carboxymethyl cellulose and/or a polysaccharide substance or a salt thereof, such as sodium carboxymethyl cellulose (Na-CMC).

11. The kit of parts for use in the treatment according to claim 9 or 10, wherein the first aqueous component comprises 2-4 wt% gel substance.

12. The kit of parts for use according to any one of the preceding claims, wherein said kit of parts comprises:
a. a first aqueous component comprising 0.1 to 1 wt% of glutamic acid, leucine and lysine and 2-4 wt% of sodium carboxymethyl cellulose, and
b. a second aqueous component comprising 1-2 wt% of sodium hypochlorite.

13. The kit of parts for use according to any one of the preceding claims, further comprising TiO₂ and/or NaCl.

14. The kit of parts for use according to any one of the preceding claims, wherein said kit of parts comprises.
a. a first aqueous component comprising:
0.4 to 0.8 wt% of glutamic acid, leucine and lysine, said amino acids being present in a relation by weight of 1:1:1,
2-4 wt% of sodium carboxymethyl cellulose,
from 0 or 0.03 to 0.1 wt % of TiO₂,
0.3 to 0.6 wt% of NaCl,
NaOH is added in an amount providing a pH from 9 to 11.5, and
water is present up to 100 wt%,
b. a second aqueous component comprising 1-2 wt% of sodium hypochlorite, NaOH is added in an amount providing a pH from 9 to 11.5, and
water is present up to 100 wt%.

15. The kit of parts for use according to any one of the previous claims, further comprising:
one or more compounds being a hydrazine derivative of formula (I)
RNHNH₂ (I)
wherein R is a chemical group containing a chromophore or forming a chromophore with NHNH₂, and/or instructions for use.

16. A treatment preparation for use in treatment and/or prevention of peri-implantitis, wherein the treatment preparation is obtainable by mixing
a. a first aqueous component as defined in any one of claims 1-14, and
b. a second aqueous component as defined in any one of claims 1-14,
wherein the first aqueous component and/or the second aqueous component has a pH from 9 to 11.5, and
wherein the treatment preparation obtainable by mixing said first aqueous component and said second aqueous component when performed at 20 degrees Celsius and at a pressure of 101325 Pa within 30 seconds after mixing has a pH from 9 to 11.5.

## Patentansprüche

1. Kit-of-Parts zur Verwendung bei der Behandlung und/oder Vorbeugung von Periimplantitis, wobei das Kit-of-Parts umfasst
a. eine erste wässrige Komponente, umfassend eine oder mehrere Aminosäuren, ausgewählt aus der Gruppe, bestehend aus Alanin, Arginin, Asparagin, Asparaginsäure, Glutaminsäure, Glutamin, Isoleucin, Leucin, Lysin und/oder Valin, und
b. eine zweite wässrige Komponente, umfassend eine aktive Chlorverbindung, ausgewählt aus der Gruppe, bestehend aus Cl₂, Hypochlorit, Chlorit, Chlorat, Perchlorat und/oder einer Hypochloritverbindung,
wobei die erste wässrige Komponente und/oder die zweite wässrige Komponente einen pH-Wert von 9 bis 11,5 aufweist, und
wobei eine Behandlungszubereitung, die durch Mischen der ersten wässrigen Komponente und der zweiten wässrigen Komponente bei 20 Grad Celsius und bei einem Druck von 101325 Pa innerhalb von 30 Sekunden nach dem Mischen erhältlich ist, einen pH-Wert von 9 bis 11,5 aufweist.

2. Kit-of-Parts zur Verwendung nach Anspruch 1, wobei das Kit-of-Parts zur Verwendung bei der Behandlung von Periimplantitis bestimmt ist.

3. Kit-of-Parts zur Verwendung nach Anspruch 2, wobei die Behandlung von Periimplantitis die chirurgische Behandlung von Periimplantitis umfasst oder aus ihr besteht.

4. Kit-of-Parts zur Verwendung nach Anspruch 1, wobei das Kit-of-Parts zur Verwendung bei der Vorbeugung von Periimplantitis bestimmt ist.

5. Kit-of-Parts zur Verwendung nach Anspruch 4, wobei die Vorbeugung von Periimplantitis die Behandlung von oraler Mukositis umfasst oder aus ihr besteht.

6. Kit-of-Parts zur Verwendung nach einem der vorstehenden Ansprüche, wobei die Menge der aktiven Chlorverbindung in der zweiten wässrigen Komponente 0,5-5, 0,5-3 oder 1-2 Gew.-% beträgt.

7. Kit-of-Parts zur Verwendung nach einem der vorstehenden Ansprüche, wobei die eine oder die mehreren Aminosäuren Glutaminsäure, Leucin und Lysin umfassen oder aus ihnen bestehen.

8. Kit-of-Parts zur Verwendung nach einem der vorstehenden Ansprüche, wobei die erste wässrige Komponente 0,1-1 Gew.-% der einen oder mehreren Aminosäuren umfasst.

9. Kit-of-Parts zur Verwendung nach einem der vorstehenden Ansprüche, wobei die erste wässrige Komponente weiter eine Gel-Substanz umfasst.

10. Kit-of-Parts zur Verwendung nach Anspruch 9, wobei die Gel-Substanz Polyethylenglykol (PEG) und/oder Carboxymethylcellulose und/oder eine Polysaccharid-Substanz oder ein Salz davon, wie Natriumcarboxymethylcellulose (Na-CMC), umfasst oder ist.

11. Kit-of-Parts zur Verwendung bei der Behandlung nach Anspruch 9 oder 10, wobei die erste wässrige Komponente 2-4 Gew.-% Gel-Substanz umfasst.

12. Kit-of-Parts zur Verwendung nach einem der vorstehenden Ansprüche, wobei das Kit-of-Parts umfasst:
a. eine erste wässrige Komponente, umfassend 0,1 bis 1 Gew.-% Glutaminsäure, Leucin und Lysin und 2-4 Gew.-% Natriumcarboxymethylcellulose, und
b. eine zweite wässrige Komponente, die 1-2 Gew.-% Natriumhypochlorit umfasst.

13. Kit-of-Parts zur Verwendung nach einem der vorstehenden Ansprüche, weiter umfassend TiO₂ und/oder NaCl.

14. Kit-of-Parts zur Verwendung nach einem der vorstehenden Ansprüche, wobei das Kit-of-Parts umfasst
a. eine erste wässrige Komponente, umfassend:
0,4 bis 0,8 Gew.-% Glutaminsäure, Leucin und Lysin, wobei die Aminosäuren in einem Gewichtsverhältnis von 1:1:1 vorliegen,
2-4 Gew.-% Natriumcarboxymethylcellulose,
0 oder 0,03 bis 0,1 Gew.-% TiO₂,
0,3 bis 0,6 Gew.-% NaCl,
NaOH wird in einer Menge zugegeben, die einen pH-Wert von 9 bis 11,5 liefert, und
Wasser ist bis zu 100 Gew.-% vorhanden,
b. eine zweite wässrige Komponente, die 1-2 Gew.-% Natriumhypochlorit umfasst,
NaOH wird in einer Menge zugegeben, die einen pH-Wert von 9 bis 11,5 liefert, und
Wasser ist bis zu 100 Gew.-% vorhanden.

15. Kit-of-Parts zur Verwendung nach einem der vorstehenden Ansprüche, weiter umfassend:
eine oder mehrere Verbindungen, die ein Hydrazinderivat der Formel (I) sind
RNHNH₂ (I)
worin R eine chemische Gruppe ist, die ein Chromophor enthält oder ein Chromophor mit NHNH₂ bildet, und/oder eine Gebrauchsanweisung.

16. Behandlungszubereitung zur Verwendung bei der Behandlung und/oder Vorbeugung von Periimplantitis, wobei die Behandlungszubereitung durch Mischen erhältlich ist,
a. einer ersten wässrigen Komponente nach einem der Ansprüche 1-14 und
b. einer zweiten wässrigen Komponente nach einem der Ansprüche 1-14, wobei die erste wässrige Komponente und/oder die zweite wässrige Komponente einen pH-Wert von 9 bis 11,5 aufweist, und
wobei die Behandlungszubereitung, die durch Mischen der ersten wässrigen Komponente und der zweiten wässrigen Komponente erhältlich ist, wenn sie bei 20 Grad Celsius und bei einem Druck von 101325 Pa innerhalb von 30 Sekunden nach dem Mischen durchgeführt wird, einen pH-Wert von 9 bis 11,5 aufweist.

## Revendications

1. Kit d'éléments pour utilisation dans le traitement et/ou la prévention de la péri-implantite, le kit d'éléments comprend
a. un premier composant aqueux comprenant un ou plusieurs acides aminés sélectionnés à partir du groupe constitué par l'alanine, l'arginine, l'asparagine, l'acide aspartique, l'acide glutamique, la glutamine, l'isoleucine, la leucine, la lysine et/ou la valine, et
b. un second composant aqueux comprenant un composé de chlore actif sélectionné à partir du groupe constitué par le Cl₂, l'hypochlorite, la chlorite, le chlorate, le perchlorate et/ou un composé d'hypochlorite,
dans lequel le premier composant aqueux et/ou le deuxième composant aqueux a un pH de 9 à 11,5 et
dans lequel une préparation de traitement susceptible d'être obtenue en mélangeant ledit premier composant aqueux et ledit deuxième composant aqueux à 20 degrés Celsius et à une pression de 101325 Pa dans les 30 secondes après le mélange, a un pH de 9 à 11,5.

2. Kit d'éléments pour utilisation selon la revendication 1, dans lequel le kit d'éléments est pour utilisation dans le traitement de la péri-implantite.

3. Kit d'éléments pour utilisation selon la revendication 2, dans lequel le traitement de la péri-implantite comprend ou consiste en un traitement chirurgical de la péri-implantite.

4. Kit d'éléments pour utilisation selon la revendication 1, dans lequel le kit d'éléments est pour utilisation dans la prévention de la péri-implantite.

5. Kit d'éléments pour utilisation selon la revendication 4, dans lequel la prévention de la péri-implantite comprend ou consiste d'un traitement de la mucosité buccale.

6. Kit d'éléments pour utilisation selon l'une quelconque des revendications précédentes, dans lequel la quantité du composé de chlore actif dans le second composant aqueux est de 0,5-5, 0,5-3 ou 1-2 % en poids.

7. Kit d'éléments pour utilisation selon l'une quelconque des revendications précédentes, dans lequel ledit ou lesdits acides aminés comprennent ou consistent d'acide glutamique, de leucine et de lysine.

8. Kit d'éléments pour utilisation selon l'une quelconque des revendications précédentes, dans lequel le premier composant aqueux comprend 0,1-1 % en poids dudit un ou plusieurs acides aminés.

9. Kit d'éléments pour utilisation selon l'une quelconque des revendications précédentes, dans lequel le premier composant aqueux comprend en outre une substance de gel.

10. Kit d'éléments pour utilisation selon la revendication 9, dans lequel la substance de gel comprend ou est du polyéthylène glycol (PEG), et/ou de la carboxyméthylcellulose et/ou une substance polysaccharidique ou un sel de ceux-ci, telle que la carboxyméthylcellulose de sodium (Na-CMC).

11. Kit d'éléments pour utilisation dans le traitement selon la revendication 9 ou 10, dans laquelle le premier composant aqueux comprend 2-4% en poids de substance de gel.

12. Kit d'éléments pour utilisation selon l'une quelconque des revendications précédentes, dans lequel ledit kit d'éléments comprend :
a. un premier composant aqueux comprenant 0,1 à 1% en poids d'acide glutamique, de leucine et de lysine et 2-4% en poids de carboxyméthylcellulose de sodium, et
b. un second composant aqueux comprenant 1-2 % en poids d'hypochlorite de sodium.

13. Kit d'éléments pour utilisation selon l'une quelconque des revendications précédentes, comprenant en outre du TiO₂ et/ou du NaCl.

14. Kit d'éléments pour utilisation selon l'une quelconque des revendications précédentes, dans lequel ledit kit d'éléments comprend :
a. un premier composant aqueux comprenant :
0,4 à 0,8 % en poids d'acide glutamique, de leucine et de lysine, lesdits acides aminés étant présents dans une relation pondérale de 1:1:1,
2-4 % en poids de carboxyméthylcellulose de sodium,
de 0 ou 0,03 à 0,1 % en poids de TiO₂,
0,3 à 0,6 % en poids de NaCI,
NaOH est ajouté en une quantité fournissant un pH de 9 à 11,5, et
l'eau est présente jusqu'à 100 % en poids,
b. un second composant aqueux comprenant 1-2 % en poids d'hypochlorite de sodium,
NaOH est ajouté en une quantité fournissant un pH de 9 à 11,5, et
l'eau est présente jusqu'à 100 % en poids.

15. Kit d'éléments pour utilisation selon l'une quelconque des revendications précédentes, comprenant en outre :
un ou plusieurs composés étant un dérivé d'hydrazine de formule (I)
RNHNH₂ (I)
dans lequel R est un groupe chimique contenant un chromophore ou formant un chromophore avec NHNH₂, et/ou un mode d'emploi.

16. Préparation de traitement pour utilisation dans le traitement et/ou la prévention de la péri-implantite, dans laquelle la préparation de traitement est susceptible d'être obtenue en mélangeant
a. un premier composant aqueux tel que défini dans l'une quelconque des revendications 1-14, et
b. un deuxième composant aqueux tel que défini dans l'une quelconque des revendications 1-14,
dans lequel le premier composant aqueux et/ou le deuxième composant aqueux a un pH de 9 à 11,5 et
dans lequel une préparation de traitement susceptible d'être obtenue en mélangeant ledit premier composant aqueux et ledit deuxième composant aqueux à 20 degrés Celsius et à une pression de 101325 Pa dans les 30 secondes suivant le mélange, a un pH de 9 à 11,5.
